(19) Europäisches Patentamt
European Patent Office
Office européen des brevets

(11) Veröffentlichungsnummer : **0 058 368**
**B1**

# (12) EUROPÄISCHE PATENTSCHRIFT

(45) Veröffentlichungstag der Patentschrift : **02.05.84**

(51) Int. Cl.³ : **C 07 C119/048**, C 07 C118/02, C 08 G 18/76

(21) Anmeldenummer : **82100893.5**

(22) Anmeldetag : **08.02.82**

## (54) Homologen- und Isomerengemische von Diisocyanaten, sowie ihre Verwendung als Aufbaukomponente bei der Herstellung von Polyurethankunststoffen.

(30) Priorität : **17.02.81 DE 3105776**

(43) Veröffentlichungstag der Anmeldung : **25.08.82 Patentblatt 82/34**

(45) Bekanntmachung des Hinweises auf die Patenterteilung : **02.05.84 Patentblatt 84/18**

(84) Benannte Vertragsstaaten : **BE DE FR GB IT**

(56) Entgegenhaltungen :
**DE-B- 1 123 662**
**DE-B- 2 063 731**

(73) Patentinhaber : **BAYER AG**
**Konzernverwaltung RP Patentabteilung**
**D-5090 Leverkusen 1 Bayerwerk (DE)**

(72) Erfinder : **Scholl, Hans-Joachim, Dr.**
**Rudolf-Sohm-Strasse 28**
**D-5000 Köln 60 (DE)**

**EP 0 058 368 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

# Homologen- und Isomerengemische von Diisocyanaten sowie ihre Verwendung als Aufbaukomponente bei der Herstellung von Polyurethankunststoffen

Die vorliegende Erfindung betrifft neue, Homologen- und Isomerengemische von Alkyl-substituierten Diisocyanaten, sowie ihre Verwendung als Aufbaukomponente bei der Herstellung von Polyurethankunststoffen nach dem Isocyanat-Polyadditionsverfahren.

Aromatische Diisocyanate sind bekannt (vgl. z. B. « POLYURETHANES », Chemistry and Technology, Part I., von Saunders & Frisch, Interscience Publishers, (1962), Seiten 17 ff.). Von großer technischer Bedeutung sind insbesondere 2,4- und 2,6-Diisocyanatotoluol bzw. beliebige Gemische dieser Isomeren (« TDI ») und 2,4'- und 4,4'-Diisocyanatodiphenylmethan bzw. beliebige Gemische dieser Isomeren, sowie Gemische dieser letztgenannten Diisocyanate mit höheren Homologen, wie sie durch Phosgenierung von Anilin/Formaldehyd-Kondensaten erhalten werden (« MDI »). Die bei Raumtemperatur flüssigen Diisocyanatotoluole sind mit dem Nachteil eines relativ hohen Dampfdruckes behaftet, was bei ihrer Verarbeitung umfangreiche Vorkehrungen erfordert, die Konzentration der Atemluft an TDI innerhalb der zulässigen Grenzen zu halten. Ein weiterer Nachteil von TDI ist in dem Umstand zu sehen, daß die Diisocyanatotoluol-isomeren nur geringfügig unterhalb Raumtemperatur liegende Schmelzpunkte aufweisen, so daß bei Lagerung und beim Transport von TDI in kalten Jahreszeiten eine zumindest teilweise Kristallisation des Diisocyanats bzw. der Diisocyanatgemische eintritt, was ein aufwendiges Aufschmelzen des Diisocyanats bzw. der Diisocyanatgemische vor ihrer Verarbeitung erforderlich macht. Die obengenannten Diisocyanate der Diphenylmethanreihe andererseits weisen zwar einen weit geringeren Dampfdruck als TDI auf, was ihre Verarbeitung in physiologischer Hinsicht weit weniger problematisch macht, jedoch sind diese Polyisocyanate mit dem Nachteil behaftet, daß das in ihnen als Hauptkomponente vorliegende 4,4'-Diisocyanatodiphenylmethan bei Raumtemperatur eine Festsubstanz darstellt, die vor ihrer Verarbeitung entweder aufgeschmolzen oder durch chemische Reaktion verflüssigt werden muß.

In der US-PS 29 34 571 wird die Herstellung von langkettigen Dinitro-alkyl-benzolen beschrieben, die gemäß US-PS 29 86 576 (entspricht DE-AS 1 123 662) über die Zwischenstufe der entsprechenden Diamine zu den entsprechenden Diisocyanaten weiterverarbeitet werden können. Bei den gemäß US-PS 29 34 571 als Ausgangsmaterial eingesetzten Alkylbenzolen handelt es sich, wie insbesondere den Ausführungsbeispielen zu entnehmen, um weitgehend definierte Verbindungen, die insbesondere eine verzweigte Oligopropylen-Seitenkette aufweisen. Zur Nitrierung dieser Kohlenwasserstoffe wird in der Vorveröffentlichung ein spezielles Nitrierverfahren vorgeschlagen, da klassische Nitierverfahren offensichtlich bei der Nitrierung der gemäß Vorveröffentlichung einzusetzenden Kohlenwasserstoffe zu den entsprechenden Dinitro-alkyl-benzolen versagen. Trotz des aufwendigen Verfahrens unter Mitverwendung von Schwefeltrioxidhaltiger Schwefelsäure entstehen beim Verfahren der US-PS 29 34 571 störende Restanteile an Mononitroverbindungen, wie eigene Untersuchungen zur Dinitrierung eines gemäß Beispiel V der Vorveröffentlichung hergestellten Dodecylbenzols belegen. Die Weiterverarbeitung dieser Zwischenstufe zu Diisocyanaten über die entsprechenden Diamine führt zwangsläufig zu Diisocyanaten mit störenden Anteile an Monoisocyanaten, die als Kettenabbrecher bei der Herstellung von Polyurethankunststoffen nach dem Isocyanat-Additionsverfahren zu unbrauchbaren Produkten führen, wie eigene Vergleichsversuche belegen. Hierdurch wird auch verständlich, daß die Diisocyanate der genannten Vorveröffentlichungen bislang keinen Eingang in die technische Polyurethanchemie finden konnten.

Es war daher die Aufgabe der vorliegenden Erfindung, neue, auf einfachem Weg zugängliche aromatische Diisocyanatgemische ohne störende Anteile an Monoisocyanaten zur Verfügung zu stellen, die einerseits einen geringeren Dampfdruck als TDI aufweisen, und die andererseits bei Raumtemperatur flüssige Substanzen darstellen, die keinerlei Tendenz zur Kristallisation aufweisen.

Diese Aufgabe konnte durch die Bereitstellung der nachstehend näher beschriebenen erfindungsgemäßen Diisocyanatgemischen gelöst werden.

Gegenstand der Erfindung sind Homologen- und Isomerengemische von Diisocyanaten der Formel

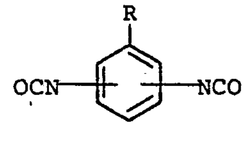

in welcher

R für einen gesättigten, geradkettigen, aliphatischen Kohlenwasserstoffrest mit 8 bis 15 Kohlenstoffatomen steht,

und welche durch Dinitrierung, anschließende Hydrierung der Nitrogruppen und Phosgenierung der so erhaltenen Aminogruppen von, als Homologengemische vorliegenden Kohlenwasserstoffen der Formel

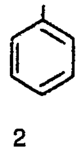

erhalten worden sind, die bei 1 013 mbar einen Siedebereich gemäß ASTM D 86 von 10-50 °C innerhalb des Temperaturbereichs von 270 °C-330 °C aufweisen.

Gegenstand der Erfindung ist auch die Verwendung der neuen Diisocyanatgemische als Aufbaukomponente bei der Herstellung von Polyurethankunststoffen nach dem Isocyanat-Polyadditionsverfahren.

Zur Herstellung der den neuen Diisocyanatgemischen zugrundeliegenden Diamine geht man von als Homologengemische vorliegenden Alkylbenzolen der Formel

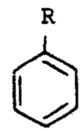

aus, welche bei 1 013 mbar einen Siedebereich gemäß ASTM D 86 von 10-50 °C, vorzugsweise 20-30 °C innerhalb des Temperaturbereichs von 270 °C-330 °C aufweisen, wobei vorzugsweise mindestens 30 Vol.-% unterhalb der Mitte des jeweiligen Siedebereichs und mindestens 30 Vol.-% oberhalb der Mitte des jeweiligen Siedebereichs sieden, und für welche

R für gesättigte, geradkettige, aliphatische Kohlenwasserstoffreste mit 8 bis 15, vorzugsweise 10 bis 13 Kohlenstoffatomen steht.

Die Herstellung derartiger Alkylbenzol-Gemische erfolgt in an sich bekannter Weise durch Alkylierung von Benzol mit den entsprechenden technischen, linearen Olefingemischen, wie beispielsweise den entsprechenden technischen Oligoethylengemischen oder anderen linearen Oligoolefinen oder mit den entsprechenden technischen, linearen Alkylchloridgemischen, wie beispielsweise in Kirk-Othmer, Encyclopedia of Chemical Technology, third edition, Vol. 2, (1978) auf Seiten 59 bis 61 beschrieben.

Zur Herstellung der einzusetzenden Diamine erfolgt zunächst die Überführung der Alkylbenzole in die entsprechenden Dinitroverbindungen durch eine an sich bekannte Nitrierungsreaktion, beispielsweise im Temperaturbereich von 10 bis 30 °C unter Verwendung von beispielsweise 2 bis 3 Mol konzentrierter Salpetersäure pro Mol Alkylbenzol, wobei die Salpetersäure in Form eines Gemisches mit konzentrierter Schwefelsäure (Nitriersäure) zur Anwendung gelangt. Bei den hierbei anfallenden Alkylsubstituierten Dinitrobenzolen sind gemäß Kernresonanzmessungen über 95 % aller Nitrogruppen in meta-Stellung zueinander angeordnet. Außerdem kann davon ausgegangen werden, daß ca. 10 bis 30 Gew.-% der dinitrierten Alkylbenzole 1-Alkyl-2,6-dinitrobenzole und ca. 70 bis 90 Gew.-% 1-Alkyl-2,4-dinitrobenzole darstellen. Diese Isomerenverteilung ist jedoch keineswegs erfindungswesentlich. So wäre es beispielsweise auch möglich, für die nächste Stufe des Verfahrens zur Herstellung der erfindungsgemäßen Ausgangsmaterialien, dinitrierte Alkylbenzole einzusetzen, in denen andere Mengenverhältnisse der genannten Isomeren vorliegen. So könnte beispielsweise für die nächste Stufe auch 1-Alkyl-2,4-dinitrobenzol in 2,6-freier Form eingesetzt werden, wie es beispielsweise durch zweistufige Nitrierung von Alkylbenzol über die Zwischenstufe des zunächst isolierten 1-Alkyl-4-nitrobenzols erhältlich ist. Überraschenderweise enthalten die aus den speziellen, obengenannten Alkylbenzolgemischen so hergestellten Dinitroverbindungen keine störenden Restanteile an Mononitroverbindungen, so daß eine Weiterverarbeitung zu Diisocyanaten ohne Anteile an Monoisocyanaten gewährleistet ist.

Die nächste Stufe besteht in der an sich bekannten Hydrierung der Nitrogruppen zu den entsprechenden primären Aminogruppen, beispielsweise unter Verwendung von Raney-Nickel als Hydrierungskatalysator bei beispielsweise 20 bis 60 °C und einem Wasserstoffdruck von beispielsweise 20 bis 40 bar.

Die so erhaltenen Diamine der oben angegebenen Formel entsprechen selbstverständlich bezüglich der Stellung der Aminogruppen den bezüglich der Stellung der Nitrogruppen gemachten Ausführungen und bezüglich des Restes R den oben bezüglich des Restes R in den als Ausgangsmaterial eingesetzten Alkylbenzolen gemachten Ausführungen.

Zur Herstellung der erfindungsgemäßen Diisocyanatgemische werden die so erhaltenen als Homologen- und Isomerengemisch vorliegenden, leicht löslichen Diamine einer an sich bekannten Phosgenierungsreaktion unterzogen. Hierzu wird beispielsweise das Diamin in einem Hilfslösungsmittel wie Chlorbenzol gelöst und in eine Lösung von Phosgen in Chlorbenzol unter Rühren und Kühlen bei − 20 bis + 5 °C, vorzugsweise − 10 bis 0 °C eingetropft (Kaltphosgenierung), wonach das Reaktionsgemisch unter fortlaufendem Rühren und Einleiten von Phosgen auf 80 bis 130 °C, vorzugsweise 90 bis 110 °C erhitzt wird, um das zunächst gebildete Carbamidsäurechlorid in das angestrebte Diisocyanat zu überführen (Heißphosgenierung). Anschließend wird das Reaktionsgemisch in an sich bekannter Weise aufgearbeitet. Selbstverständlich kann die Überführung der Diamine in die erfindungsgemäßen Diisocyanate auch nach beliebigen anderen, an sich bekannten Phosgeniermethoden erfolgen.

Die erfindungsgemäßen Diisocyanatgemische stellen flüssige Substanzen dar, die keinerlei Kristallisationsneigung erkennen lassen, die, bedingt durch ihren geringen Dampfdruck, bei Raumtemperatur keinen nennenswerten Eigengeruch aufweisen, und die keine störenden Restanteile an Monoisocyanaten aufweisen. Es handelt sich um Homologen- und Isomerengemische von Diisocyanaten der obengenannten allgemeinen Formel, wobei bezüglich der Stellung der Isocyanatgruppen die oben gemachten Ausführungen bezüglich der Stellung der Nitrogruppen und bezüglich des Restes R die oben gemachten Ausführungen bezüglich des Restes R in den als Ausgangsmaterial eingesetzten Alkylbenzolen Gültigkeit haben. Die erfindungsgemäßen Diisocyanatgemische stellen wertvolle Ausgangsmaterialien zur Herstellung von Polyurethankunststoffen nach dem Isocyanat-Polyadditionsverfahren dar. Für diesen Verwendungszweck können die erfindungsgemäßen Diisocyanate anstelle der bislang eingesetzten aromatischen Diisocyanate eingesetzt werden. Dies bedeutet ; daß sich die erfindungsgemäßen Diisocyanate hervorragend zur Herstellung von beispielsweise Polyurethanschaumstoffen, -elastomeren, -klebstoffen, -dispersionen, -beschichtungen oder -lacken nach den an sich bekannten Verfahren unter Mitverwendung der an sich bekannten Reaktionspartner und Hilfsstoffe eignen. Die erfindungsgemäßen Diisocyanate stellen außerdem wertvolle Zwischenprodukte zur Herstellung von Schädlingsbekämpfungsmitteln dar.

Der erfindungswesentliche Punkt ist darin zu sehen, daß erst aufgrund der erfindungsgemäß getroffenen Auswahl bezüglich des als Homologengemisch vorliegenden Alkylbenzols durch eine an sich bekannte Nitrierungs-, Hydrierungs- und Phosgenierungsreaktion Monoisocyanat-freie, flüssige, leicht lösliche Diisocyanatgemische mit niedrigem Dampfdruck und ohne Kristallisationsneigung in technisch einfacher Weise erhalten werden können.

Die nachfolgenden Beispiele dienen der näheren Erläuterung der Erfindung. Alle Prozentangaben beziehen sich, falls nicht anders lautend erwähnt, auf Gewichtsprozente.

Für die dünnschichtchromatographischen Untersuchungen wurden DC-Fertigplatten, Kieselgel 60 F-254 der Firma Merck AG verwendet.

## Beispiel 1

In diesem Beispiel wird ein Homologengemisch linearer Alkylbenzole eingesetzt, deren Alkylketten eine Länge von 10 bis 13 C-Atomen bei einer mittleren Kettenlänge von etwa 12 C-Atomen aufweisen. Das Homologengemisch siedet gemäß ASTM D 86 bei 1 013 mbar zwischen ca. 283 und 313 °C, wobei ca. 50 Vol.% bis 296 °C übergehen. Es handelt sich um ein Umsetzungsprodukt von Benzol mit einem Gemisch aus linearen $C_{10}$-$C_{13}$-Olefinen.

a) Nitrierung des Alkylbenzolgemischs :

In 1,97 kg Alkylbenzolgemisch wird eine Mischung von 1 136 ml 98 %iger Salpetersäure und 1 584 ml 96 %iger Schwefelsäure unter Kühlen so eingetropft, daß die Innentemperatur bei 10 bis 15 °C liegt. Nach Beendigung des Zutropfens wird 3 Stunden bei 25 bis 30 °C nachgerührt. Das Reaktionsgemisch wird nachfolgend auf 10 kg Eis geschüttet, die organische Phase mit Natriumbicarbonatlösung neutral gewaschen und nochmals mit Wasser nachgewaschen. Die organische Phase wird abgetrennt und von verbleibenden Wasseranteilen durch Zentrifugieren weitgehend befreit. Das so erhaltene, flüssige Dinitroalkylbenzolgemisch wurde ohne weitere Reinigung der nächsten Reaktionsstufe zugeführt.

Ausbeute : 2,7 kg ; $NO_2$-Gehalt : 27,5 % (Theorie : 27,4 %). Nach dünnschichtchromatographischen Untersuchungen waren keine Restanteile an Mononitroverbindungen nachzuweisen (Laufmittel 90 Gew.-Teile Petrolether/10 Gew.-Teile Ether).

b) Hydrierung des Dinitroalkylbenzolgemischs :

In einem Rührautoklaven werden 672 g der Dinitroverbindung gemäß a) in 1 700 ml Ethanol gelöst und mit 70 g Raney-Nickel versetzt. Bei 40 °C und 20 bis 40 bar Wasserstoffdruck wird bis zum Ende der Wasserstoffaufnahme gerührt. Anschließend wird entspannt, vom Katalysator abfiltriert und das Ethanol abdestilliert. Man erhält 550 g Rohamingemisch, welches ohne destillative Aufarbeitung der Stufe c) zugeführt werden kann. Das Rohamingemisch weist einen Stickstoffgehalt von 10,06 % (Theorie : 10,1 %) auf.

c) Phosgenierung des Rohamingemischs :

In einem 6 l Vierhalskolben, mit Rührer, Thermometer, Gaseinleitungsrohr und Rückflußkühler versehen, werden 3 l trockenes Chlorbenzol vorgelegt. Unter Rühren und Kühlung (– 10 °C) werden ca. 800 g Phosgen einkondensiert, anschließend läßt man unter Kühlung bei – 10 bis – 5 °C 550 g Rohamingemisch gemäß Stufe b), in 500 ml Chlorbenzol gelöst, zutropfen. Unter weiterem Einleiten von Phosgen erwärmt sich die Lösung anschließend ohne weitere Kühlung auf ca. 30 °C. Nach Abklingen der Wärmetönung wird langsam bis 100 °C aufgeheizt und bis zum Ende der Chlorwasserstoffentwicklung Phosgen eingeleitet (insgesamt 970 g). Das überschüssige Phosgen wird mit Stickstoff ausgeblasen und die Lösung im Vakuum eingedampft. Man erhält 638 g Rohisocyanatgemisch mit einem NCO-Gehalt von 25 % (Theorie : 25,6 %). Das so erhaltene erfindungsgemäße Diisocyanat kann ohne weitere Reinigung als Ausgangsmaterial zur Herstellung von Polyurethanen verwendet werden, oder aber einer zusätzlichen destillativen Reinigung unterworfen werden :

300 g des Diisocyanatgemisches werden unter vermindertem Druck destilliert. Bei einem Druck von 2,2 mbar destillieren innerhalb des Temperaturbereichs von 185 bis 203 °C 273 g eines praktisch farblosen Gemischs von Diisocyanaten der Formel

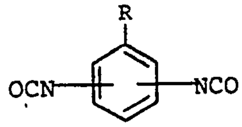

welches auch bei Abkühlung auf – 50 °C keinerlei Tendenz zur Kristallbildung erkennen läßt.

Analyse (%):

| | NCO | C | H | N |
|---|---|---|---|---|
| gefunden: | 25,5 | 73,6 | 8,6 | 8,4 |
| Theorie: (bezogen auf $C_{20}H_{28}N_2O_2$) | 25,6 | 73,2 | 8,5 | 8,5 |

Nach Kernresonanzmessungen sind die Isocyanatgruppen des Diisocyanats in meta-Stellung zueinander angeordnet. Die Hauptkomponente des Gemischs besteht aus 1-Alkyl-2,4-diisocyanatobenzol.

Beispiel 2 (Vergleichsbeispiel)

a) Durch Nacharbeitung von Beispiel V der US-PS 2 934 571 und der Beispiele V und VI von US-PS 2 986 576 wurde ein Dodecyl-phenylen-diisocyanat hergestellt. Der ermittelte NCO-Gehalt dieses so hergestellten Diisocyanats lag bei 20,2 %.

Der geringe NCO-Gehalt ist auf die unvollständig ablaufende Dinitrierung des verwendeten Dodecylbenzols zurückzuführen. Dünnschichtchromatographische Untersuchungen zeigen erhebliche Anteile an Mononitroverbindungen an.

b) Mit dem erfindungsgemäßen Diisocyanatgemisch gemäß Beispiel 1 und dem Diisocyanat gemäß 2a) wurden jeweils ein Präpolymer hergestellt :

0,25 Mol eines Polyesters der OH-Zahl 45, hergestellt aus Adipinsäure und Diethylenglykol und 0,5 Mol Diisocyanat wurden jeweils bei 80 °C bis zum konstanten NCO-Gehalt des Reaktionsgemischs umgesetzt. Die Präpolymere wurden jeweils in DMF-Toluol (1 : 1) zu 65 %igen Lösungen gelöst und anschließend unter Rühren bei Raumtemperatur tropfenweise mit ca. 42 g IPDA versetzt ($NCO/NH_2$-Äquivalentverhältnis = ca. 1 : 1). Nach Verschwinden der NCO-Bande im IR-Spektrum wurde durch Zugabe von Isopropanol auf eine 50 %ige Lösung in DMF/Toluol/Isopropanol eingestellt.

Die Lösung des so hergestellten Polyurethan-Polyharnstoffs unter Verwendung des erfindungsgemäßen Diisocyanatgemischs gemäß Beispiel 1 wies eine Viskosität von 25 000 mPa.s/20 °C auf. Der aus der Lösung hergestellte, lösungsmittelfreie Film des Polyurethan-Polyharnstoffs ist trocken, nicht klebrig und weist eine Zugfestigkeit von 10,4 mPa auf. Die Lösung des Polyurethan-Polyharnstoffs, die unter Verwendung des Diisocyanats gemäß 2a) hergestellt worden war, wies demgegenüber eine Viskosität von lediglich 400 mPa.s/20 °C auf. Der Versuch, aus dieser Lösung einen trockenen Film herzustellen, führte zu einer schmierigen, verfließlichen Beschichtung, die keinerlei mechanische Festigkeit aufwies.

Beispiel 3 (Verwendungsbeispiel)

Das nachfolgende Beispiel zeigt die Verwendung des erfindungsgemäßen Diisocyanatgemischs gemäß Beispiel 1 zur Herstellung eines Polyurethan-Zweikomponentenlackes und zeigt, daß durch die besonderen Eigenschaften des neuen Diisocyanates die Anwendung dieses Lackes vorteilhaft in einem Lösungsmittel erfolgen kann, das zu einem hohen Anteil aus aliphatischen Kohlenwasserstoffen besteht.

Zur Herstellung des Lackes benutzt man als Hydroxylgruppen tragendes Bindemittel ein Alkydharz, das aus

1,6 Mol Phthalsäureanhydrid,
2,7 Mol Trimethylolpropan,
0,6 Mol Adipinsäure,
1,35 Mol Sojaölfettsäure (mittleres Molgewicht ca. 290) und
0,16 Mol Maleinsäure

besteht und durch übliche Schmelzkondensation hergestellt wurde.

Dieses Polyesterharz hat eine Hydroxylzahl von 110 und eine Säurezahl von 7 und wird 50 %ig in einem Gemisch von Lackbenzin/Xylol 5 : 2 aufgelöst. (Die Bezeichnung Lackbenzin bedeutet ein der Lösungsmittelverordnung vom 26.02.1954 entsprechendes Testbenzin, das nicht als gesundheitsschädlich zu kennzeichnen ist.)

Aus dem Polyesterharz wird mit verschiedenen Hilfsprodukten ein Lack folgender Zusammensetzung hergestellt :

| | |
|---|---|
| Polyesterharz (50 %ig in Benzin/Xylol 5 : 2) | 300,0 Gewichtsteile |
| $TiO_2$-Pigment (Rutil-Typ) | 90,0 Gewichtsteile |
| Zinkoctoat (8 % Metall) als Katalysator | 0,6 Gewichtsteil |
| Hautverhinderungsmittel (Butanonoxim) | 1,5 Gewichtsteile |
| Silicon-Verlaufsmittel 1 %ig | 4,0 Gewichtsteile |

Die Bestandteile werden vermischt und das Pigment über eine Sandmühle eingearbeitet.

Zu der Anreibung gibt man unter guter Vermischung eine 50 %ige Lösung von 49 Teilen des erfindungsgemäßen Isocyanatgemisches aus Beispiel 1 in Lackbenzin (entspricht einem Verhältnis von OH : NCO von etwa 1).

Der Lackansatz hat eine Verarbeitungszeit von mehr als 24 Stunden, in der sich die Viskosität praktisch nicht verändert. Danach steigt die Viskosität langsam an, dabei bleibt der Lackansatz aber einheitlich, ohne daß sich Quellkörper oder Niederschläge bilden und läßt sich nach Verdünnen mit Lackbenzin erneut verarbeiten.

Der Lack wird mit einer Lackhantel bzw. einer airless-Spritzanlage in einer Naßschichtdicke von ca. 100 μ auf entfettete Stahlbleche aufgetragen und ausgehärtet.

Man erhält bei einer Trocknung von 20-30 Min. bei 80 °C reinweiße, hochglänzende Lackfilme. Sie haben eine ausgezeichnete Lösungsmittelbeständigkeit (5 Min. Einwirkung von Toluol-keine Veränderung), hohe Elastizität (Erichsen-Tiefung DIN 53 156-100 mm/Blechriß) und eine ausreichende Härte (Pendelhärte DIN 53 157-160 Sec.).

Im Vergleich zu diesem Lack mit dem erfindungsgemäßen Isocyanat-Gemisch wurde versucht in den gleichen Ansatz die entsprechenden molaren Mengen 4,4'-Diphenylmethan-diisocyanat und ein Gemisch (80 : 20) von 2,4- und 2,6-Diisocyanatotoluol einzuarbeiten. Mit 4,4'-Diphenylmethandiisocyanat ist das nicht möglich, da sich dieses Diisocyanat nur in der Wärme in Lackbenzin auflöst und in Kombination mit der Polyesterlösung eine inhomogene Mischung bildet, aus der sich nach kurzer Zeit ein Niederschlag abscheidet.

Diisocyanatotoluol läßt sich zwar mit dem Polyester homogen vermischen, bildet aber ebenfalls nach kurzer Zeit eine starke Trübung.

Die Klarlackfilme sind nach Trocknung bei 80 °C inhomogen, die mit $TiO_2$ pigmentierten Filme haben keinen Glanz und sind matt. Abgesehen von diesem Nachteil, ist Diisocyanatotoluol aufgrund seines hohen Dampfdrucks und unangenehmen Geruchs nicht für 2-Komponentenlacke geeignet.

Fügt man dem Diisocyanatotoluol enthaltenden 2-Komponentenlack anstelle von Benzin ein polares Lösemittel wie Ethylenglykolacetat zu, so lassen sich auch hier glänzende Lackfilme erhalten.

**Ansprüche**

1. Homologen- und Isomerengemische von Diisocyanaten der Formel

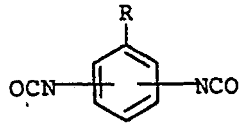

in welcher

R für einen gesättigten, geradkettigen, aliphatischen Kohlenwasserstoffrest mit 8 bis 15 Kohlenstoffatomen steht,

und welche durch Dinitrierung, anschließende Hydrierung der Nitrogruppen und Phosgenierung der so erhaltenen Aminogruppen von als Homologengemisch vorliegenden Kohlenwasserstoffen der Formel

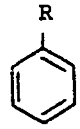

erhalten worden sind, die bei 1 013 mbar einen Siedebereich gemäß ASTM D 86 von 10-50 °C innerhalb des Temperaturbereichs von 270 °C-330 °C aufweisen.

2. Verwendung der Homologen- und Isomerengemische gemäß Anspruch 1 als Aufbaukomponente bei der Herstellung von Polyurethankunststoffen nach dem Isocyanat-Polyadditionsverfahren.

**Claims**

1. Mixtures of homologues and isomers of diisocyanates of the formula

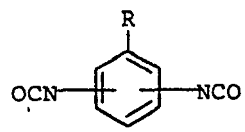

in which

R represents a saturated, straight-chain, aliphatic hydrocarbon radical with 8 to 15 carbon atoms, and which have been obtained by dinitration, subsequent hydrogenation of the nitro groups and phosgenation of the amino groups thus obtained, of hydrocarbons, present as a homologue mixture, of the formula

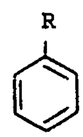

which, at 1 013 mbar, have a boiling range according to ASTM D 86 of 10-50 °C within the temperature range of 270 °C-330 °C.

2. Use of the mixtures of homologues and isomers according to Claim 1 as a starting component in the production of polyurethane plastics by the isocyanate polyaddition process.

**Revendications**

1. Mélanges d'homologues et d'isomères de diisocyanates de formule

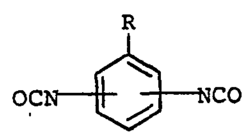

dans laquelle

R représente un radical d'hydrocarbure aliphatique saturé à chaîne droite contenant 8 à 15 atomes de carbone,

ces mélanges étant obtenus par dinitration, puis par hydrogénation des groupes nitro et par phosgénation des groupes amino ainsi obtenus d'hydrocarbures présents sous forme de mélanges d'homologues, répondant à la formule

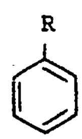

et ayant, à 1 013 mbars, un intervalle d'ébullition (suivant la norme ASTM D 86) de 10-50 °C, à une température se situant dans l'intervalle de 270 à 330 °C.

2. Utilisation des mélanges d'homologues et d'isomères suivant la revendication 1 comme composants structuraux lors de la fabrication de matières synthétiques de polyuréthanes selon le procédé de polyaddition d'isocyanates.